# EUROPEAN PATENT APPLICATION

(11) **EP 3 192 558 A1**
(43) Date of publication of application: **19.07.2017**
(21) Application number: 16151367.6
(22) Date of filing: 14.01.2016
(51) Int. Cl.: A61N 1/04

(54) **SHEET MASK FOR IONTOPHORESIS AND MASK ASSEMBLY USING THE SAME**

(71) Applicant: Insung Information Co., Ltd., Seoul 138-855 (KR); Womans Care Co., Ltd., Gyeonggi-do, Goyang-si 410-315 (KR)
(72) Inventor: CHUN, Young Sam, 413-130 Paju-si (KR); PARK, Il Ju, 400-340 Incheon (KR); KIM, Hong Jin, 138-855 Seoul (KR); SHIN, Moo Kyung, 420-813 Bucheon-si (KR); KIM, Myoung Hwan, 122-852 Seoul (KR)
(74) Representative: Zardi, Marco

(57) **Abstract**

The present invention relates to a sheet mask for iontophoresis and, more specifically, to a sheet mask for iontophoresis including first boring portions formed at portions corresponding to eyes, a nose, and a mouth of a user face, and second boring portions formed to connect electrode portions.

## Description

### BACKGROUND OF THE INVENTION

### Field of the Invention

The present invention relates to a sheet mask for iontophoresis and a mask assembly using the same and, more specifically, to a sheet mask for iontophoresis including first boring portions formed at portions corresponding to eyes, a nose, and a mouth of a user face, and second boring portions formed to connect electrode portions, and a mask assembly using the same.

### Description of the Related Art

Generally, in case of transcutaneously administering an ionic, weak ionic, or non-ionic drug, a method of administering an effective drug into a human body through a skin or mucous membrane by using characteristics that ions of drug molecules or solvent molecules electric-currently move through electrical propulsive force is mainly used to induce rapid and much administration thereof. For example, there are iontophoresis, electroosmosis, electroporation, and the like, and there are also sonophoresis and laserporation using sound waves and light beams that are physical means.

As means using such electrical propulsive force, iontophoresis has been most representatively known, and the iontophoresis means a method in which micro-current is allowed to flow on a skin such that a drug or an effective component having charges infiltrates into the skin by electrical repulsive force. In order that a drug or an effective component infiltrates into a biological tissue by electric current, electrical repulsive force of an ionized drug is used. In the ionized drug ions (+ or -), according to polarity of electric current, cations (+) having high mobility move to a cathode electrode (-), anions (-) move to an anode electrode (+), and thus the drug ions (+ or -) having electrostatic charges are administered to the skin. As a method of increasing material absorption into a skin, there is a method of changing physical and chemical properties using an absorbefacient or a prodrug such that infiltration of a drug is promoted, but iontophoresis is a method of even more actively increasing drug infiltration than the existing method by forcibly changing electrical environment of a skin, and is also a drug administration method which is even less invasive than injection. Particularly, a method of absorbing vitamin C that is a skin whitening efficacy material to a skin using iontophoresis to mitigate freckles is being currently used in dermatology hospitals, and the method is known as having a significant effect in treatment of freckles.

### Citation List

### Patent Literature

Patent Literature 1: KR 10-1198690 B1

### SUMMARY OF THE INVENTION

Currently, when iontophoresis is applied to a skin, charges charged on the skin are accumulated and polarized due to the use of direct current. In addition, as for direct current EH, when alternating current is applied to an anode electrode and a cathode electrode, an electrical operation occurs in a living body, acidity of a skin under the anode electrode becomes high, and basicity becomes high on a skin on the cathode electrode side. Particularly, since concentration of a base acting on the cathode electrode is higher, skin resistance is decreased, the amount of current is increased, and thus larger damage is caused on the skin on the cathode electrode side. In the case of lidocain drug injection using the iontophoresis system, it is known that damage is further represented on a skin and a mucous membrane on the cathode electrode side.

The invention has been contrived to solve the problems, and is to provide a sheet mask for iontophoresis including first boring portions which are formed at positions corresponding to eyes, a nose, and a mouth of a user face, and second boring portions which are formed to connect electrode portions, wherein one to six second boring portions are formed at an edge portion or separately protruding portions of the sheet mask, and further including third boring portions for fitting to ears of a user. In addition, a mask assembly using the same is provided.

### BRIEF DESCRIPTION OF THE DRAWINGS

FIG. 1 is a plan view of a sheet mask for iontophoresis in which second boring portions and third boring portions are formed at an edge portion of the sheet mask according to a first embodiment of the invention;
FIG. 2 is a plan view of a sheet mask for iontophoresis in which second boring portions are formed at separately protruding portions of the sheet mask and third boring portions are formed at an edge portion of the sheet mask according to a second embodiment;
FIG. 3 is a plan view of a sheet mask for iontophoresis in which second boring portions are formed at an edge portion of the sheet mask and third boring portions are formed at separately protruding portions of the sheet mask according to a third embodiment;
FIG. 4 is a plan view of a sheet mask for iontophoresis in which second boring portions and third boring portion are formed at separately protruding portions of the sheet mask according to a fourth embodiment; and
FIG. 5 is a plan view of a sheet mask in which first boring portions formed at portions corresponding to eyes and a nose has an open-close shape formed only by predetermined incisions.

### DETAILED DESCRIPTION OF THE PREFERRED EMBODIMENTS

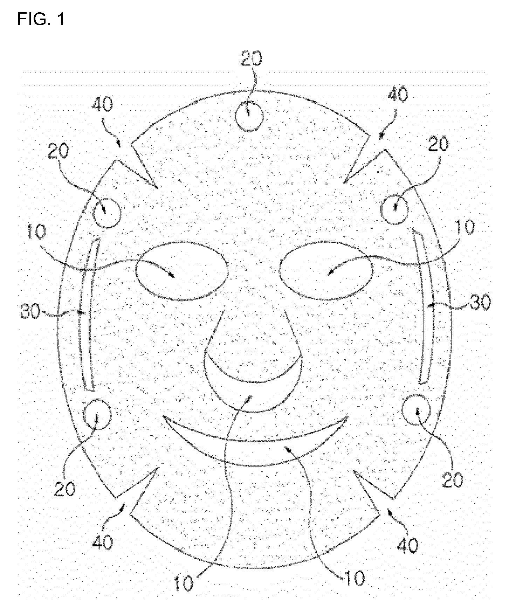

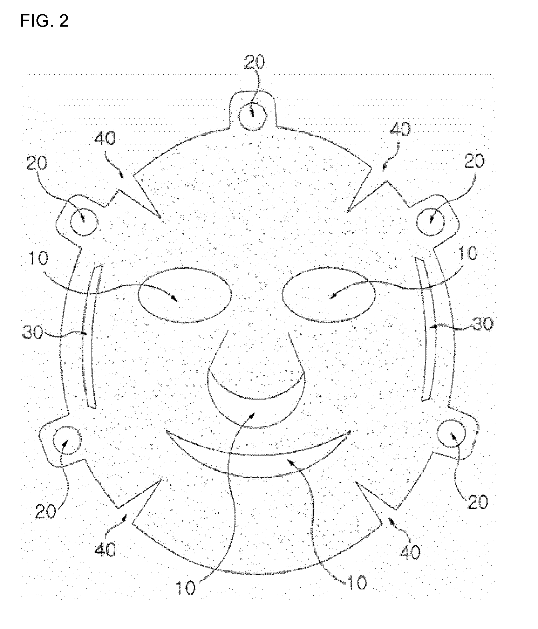

The present invention relates to a sheet mask for iontophoresis and, more specifically, to a sheet mask for iontophoresis including first boring portions 10 which are formed at portions corresponding to eyes, a nose, and a mouth of a user face, and second boring portions 20 which are formed to connect electrode portions. An embodiment of the invention is illustrated in FIG. 1 and FIG. 2, and the invention will be described in detail with reference thereto.

The sheet mask for iontophoresis of the invention is manufactured with a predetermined size to cover user's face, and may be manufactured with a material having predetermined elasticity of coming in close contact with three-dimensional user's face and absorptivity containing liquid essence.

### Shape of Sheet Mask

### 1) First Boring Portion

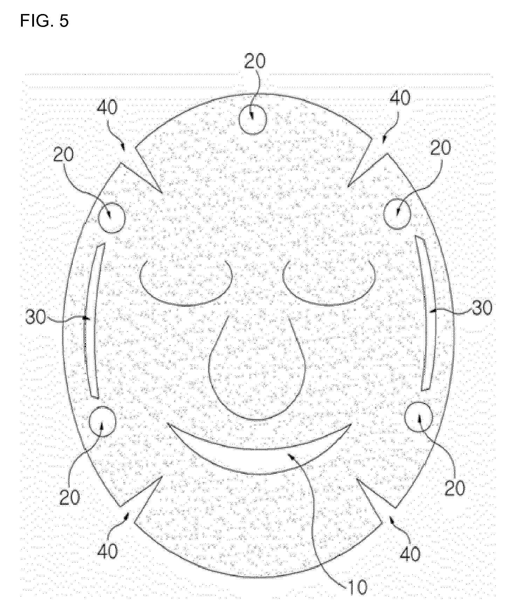

A sheet mask for iontophoresis of the invention includes first boring portions 10 which are formed at least one portion of portions corresponding to eyes, a nose, and a mouth of a user face. Particularly, the first boring portion 10 formed at the portion corresponding to a nose may be formed including a predetermined incision as illustrated in FIG. 1 to FIG. 4, or may be formed with an incision which is not bored as illustrated in FIG. 5. In addition, the first boring portions 10 formed at the portions corresponding to eyes may have an open-close shape formed only with predetermined incisions as illustrated in FIG. 5, and the shape of the first boring portion is not limited, but the boring portion should be formed at at least one portion.

### 2) Second Boring Portion

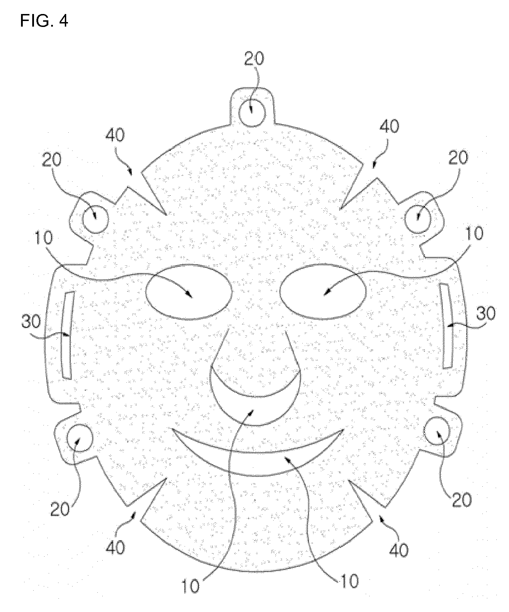

The sheet mask for iontophoresis of the invention includes one to six second boring portions 20 for connecting electrode portions. The second boring portions 20 have a predetermined size, and may be formed at an edge portion of the sheet mask as illustrated in FIG. 1, FIG. 3, and FIG. 5, or may be formed at separately protruding portions as illustrated in FIG. 2 and FIG. 4.

As described above, the second boring portions 20 formed at the edge portion or separately protruding portions of the sheet mask may be formed to be adjacent to both portions around eyes, both portions around a mount, or a forehead portion and, preferably, the second boring portions 20 may be connected to cathode or anode electrode portions divided into two portions. In clinical trials of the invention, a sheet mask in which a cathode electrode is divided into two portions and which contains a vitamin C component is applied and, as a result of confirmation of a whitening effect according thereto, it was confirmed that the effect is more excellent as a skin portion gets closer to the electrode portion. Accordingly, it is preferable to dispose the electrode portions symmetrically with respect to a portion for a concentrated effect.

### 3) Third Boring Portion

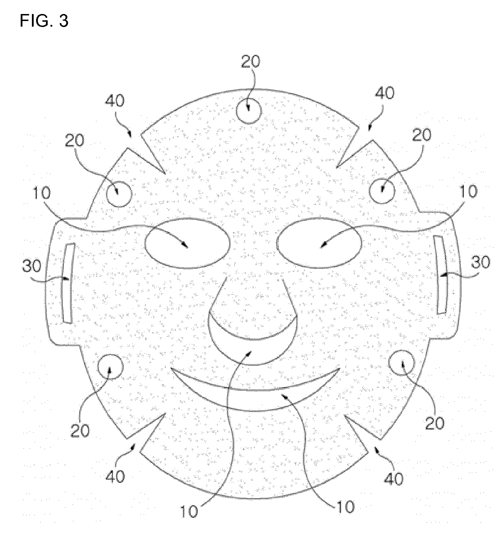

In the sheet mask for iontophoresis of the invention, third boring portions 30 for fitting to user's ears are further formed in addition to the first boring portions 10 and the second boring portions 20, and it is possible to fix the sheet mask onto a user face at the time of using. The third boring portions 30 may be also formed at the edge portion or separately protruding portions of the sheet mask similarly to the second boring portions (20), FIG. 1, FIG. 2, and FIG. 5 illustrate a shape in which the third boring portions 30 are formed at the edge portion of the sheet mask, and FIG. 3 and FIG. 4 illustrate a shape in which the third boring portions 30 are formed at the separately protruding portions of the sheet mask.

### 4) Notch Portion

In the sheet mask for iontophoresis of the invention, a plurality of notch portions 40 for close contact with a user face may be formed, the shape thereof is a shape including an incision, and there is no limit in shape and position.

### Component included in the Sheet Mask

In the sheet mask for iontophoresis of the invention, an essence component is uniformly applied and impregnated, and the essence may contain a functional component of iontophoresis to a user skin. Particularly, the functional component may be liquid vitamin C known as whitening and antioxidant effects, and may include a vitamin C component of 0.1 to 10 parts by weight with respect to 100 parts by weight of the essence entirely applied to and impregnated in the sheet mask.

In addition thereto, humectant, surfactant, preservative, purified water, and the like may be further included at a predetermined ratio. The humectant acts as a water retention agent thereof, and also plays an important role of contributing even to maintenance of stability of a system. Polyvalent alcohol of trivalent or more such as glycerin or sorbitol has an excellent moisturizing property and water retention property, and is used as the highest general-purpose humectant in terms of safety, stability, price, and the like. As necessary conditions provided as humectants, it does not depend on environment conditions (temperature, humidity, wind, and the like), particularly, it does not depend on an ambient temperature, and it is possible to keep moisture for a long time. The surfactant is non-ionic surfactant excellent in safety such as hypoallergenic, and the known thing may be used such as polyglycerol fatty acid ester that is an esterified compound of polyglycerine and fatty acid.

### How to Use the Sheet Mask

In general iontophoresis, charges charged in a skin are accumulated and polarized due to the use of direct current and, in the iontophoresis of the invention to overcome it, it is possible to discharge charges accumulated according to a pulse time ratio by using pulse current. Accordingly, various studies about an electrical connection device and method between an anode electrode and a cathode electrode using pulse current have been made. For example, in Japanese Patent Publication No. 2-45461, a pulse depolarization type method is disclosed, and this method actively discharges charges accumulated between an anode electrode and a cathode electrode.

In addition, as a cause of skin damage based on direct/alternating current, there is change in pH concentration in a living body. When the direct/alternating current is applied to the anode electrode and the cathode electrode, an electrical operation occurs in the living body, acidity of a skin under the anode electrode becomes high, and basicity becomes high on a skin on the cathode electrode side. Particularly, since concentration of a base acting on the cathode electrode is higher, skin resistance is decreased, the amount of current is increased, and thus larger damage is caused on the skin on the cathode electrode side. In the case of lidocain drug injection using the iontophoresis system, it is known that damage is further represented on a skin and a mucous membrane on the cathode electrode side.

Accordingly, the electrode portions connected to the second boring portions 20 have a shape divided into at least two parts, and it is preferable to separate electrical stimulation positions of a skin using a pulse-type current keeping a current cutoff period of direct current to regular intervals. Stable pulse current is applied to a skin and a mucous membrane, a skin adverse effect electrically operated on the cathode electrode is suppressed, and thus it is possible to reduce damage of a skin and a mucous membrane occurring in the existing iontophoresis device.

### Material of the Sheet Mask

A material of the sheet mask for iontophoresis of the invention may be non-woven fabric, and it is preferable that the non-woven fabric is manufactured using pulp, synthetic fiber (viscos rayon), or chemical fiber (PET, PP, nylon, etc.) to have excellent touch and flexibility, good breathability and filtration function, and harmlessness of a human body.

A material of the sheet mask for iontophoresis may be a gel type, and may be applied by mixing locust bean gum that is a food additive for increasing adhesiveness and viscosity of food, increasing emulsion stability, and improving physical property and touch of food, and carrageenan that is a food additive for increasing adhesiveness and viscosity of food, increasing emulsion stability, and improving physical property and touch of food, with water, to form a gelled composition in a gel state having viscosity, in a mask form.

In addition, a mask assembly for iontophoresis of the invention will be described. The mask assembly may be configured including the sheet mask described above, electrode portions (each set of cathode electrodes and anode electrodes) which are installed at the second boring portions and have a function of generating current flow in the mask, and a power supply unit. In terms of current flow, electric current supplied from an external power source flows through a cable and the electrode portion (cathode electrode) to the entire surface of the mask, electric current entering from the mask into a face skin flows into another electrode (ground electrode, anode electrode) attached to the other portion of a body such as a neck, shoulders, or arms, and finally, the electric current flows on one closed circuit, thereby embodying ion injection.

In the sheet mask for iontophoresis according to the invention, one to six second boring portions formed to connect the electrode portions are formed, the electrode portions have a shape divided into at least two, and it is possible to expect a symmetrical uniform effect of a functional product by connecting the electrode portions to selective positions. In addition, the electrical stimulation positions of a skin are separated using pulse-type current keeping a current cutoff period of alternating current to regular intervals, to apply stable pulse current to a skin and a mucous membrane and to suppress a skin adverse effect electrically acting on the cathode electrode, and thus it is possible to reduce damage of a skin and a mucous membrane occurring in the existing iontophoresis device.

The invention has been described with reference to the accompanying drawings, but it is merely one embodiment of various embodiments including the gist of the invention, an object thereof is to allow a person skilled in the art to easily embody the invention, and it is clear that the invention is not limited only the embodiments described above. Accordingly, the protective scope of the invention should be interpreted by the following Claims, and all the technical spirits falling within the scope equivalent to modification, replacement, and substitution within the scope which does not deviate from the gist of the invention should be included in the right scope of the invention. In addition, some configurations of the drawings are to clearly describe the configurations, and it is clear that they are provided by exaggeration and reduction as compared with actual things.

## Claims

1. A sheet mask for iontophoresis comprising:
a first boring portion which is formed at least one portion of portions corresponding to eyes, a nose, and a mouth of a user face; and
a second boring portion which is formed to connect an electrode portion,
wherein the number of second boring portions is one to six.

2. The sheet mask for iontophoresis according to Claim 1, wherein the second boring portion is formed at an edge portion of the sheet mask.

3. The sheet mask for iontophoresis according to Claim 1, wherein the second boring portion is formed at a separately protruding portion of the sheet mask.

4. The sheet mask for iontophoresis according to Claim 2 or 3, wherein the second boring portions are formed to be adjacent to both portions around eyes.

5. The sheet mask for iontophoresis according to Claim 2 or 3, wherein the second boring portions are formed to be adjacent to both portions around a mouth.

6. The sheet mask for iontophoresis according to Claim 2 or 3, wherein the second boring portion is formed around a forehead.

7. The sheet mask for iontophoresis according to Claim 1, further comprising third boring portions for fitting to ears of a user.

8. The sheet mask for iontophoresis according to Claim 1, further comprising a plurality of notch portions for close contact with a user face.

9. The sheet mask for iontophoresis according to Claim 1, wherein essence is applied onto the sheet mask, and the essence contains a functional component of iontophoresis to a user skin.

10. The sheet mask for iontophoresis according to Claim 9, wherein the functional component is liquid vitamin C, and 100 parts by weight of the essence entirely applied onto the sheet mask includes 0.1 to 10 parts by weight of a vitamin C component.

11. The sheet mask for iontophoresis according to Claim 1, wherein the number of electrode portions connected to the second boring portions is at least two, and electrical stimulation positions of a skin are separated using a pulse-type current keeping a current cutoff period of direct current to regular intervals.

12. A mask assembly for iontophoresis comprising:
the sheet mask according to any one of Claims 1 to 3 and Claims 7 to 11;
an electrode portion which is installed in the second boring portion and has a function of generating current flow in the mask; and
a power supply unit which supplies power to the electrode portion.
